# EUROPÄISCHE PATENTSCHRIFT

(11) **EP 2 828 559 B1**
(45) Veröffentlichungstag und Bekanntmachung des Hinweises auf die Patenterteilung: **19.10.2016**
(21) Anmeldenummer: 13710833.8
(22) Anmeldetag: 15.03.2013
(51) Int. Cl.: F16J 15/02, F16J 15/06

(54) **MEDIZINISCHE DICHTUNG UND MEDIZINISCHER STERILISIERBEHÄLTER**
MEDICAL SEAL AND MEDICAL STERILISATION VESSEL
JOINT MÉDICAL ET RÉCIPIENT STÉRILE MÉDICAL

(30) Priorität: 20.03.2012 DE 102012102370
(43) Veröffentlichungstag der Anmeldung: 28.01.2015
(73) Patentinhaber: Aesculap AG, 78532 Tuttlingen (DE)
(72) Erfinder: WEISSHAUPT, Dieter, 78194 Immendingen (DE); SCHUSTER, Stefan, 78048 Villingen-Schwenningen (DE); THOMAS, Stefan, 78532 Tuttlingen (DE); AICHER, Gerhard, 78600 Kolbingen (DE); ZIERIS, Gerold, 78570 Mühlheim (DE)
(74) Vertreter: Hoeger, Stellrecht & Partner Patentanwälte mbB
(86) Internationale Anmeldenummer: PCT/EP2013/055312
(87) Internationale Veröffentlichungsnummer: WO 2013/139682

(56) Entgegenhaltungen:
- EP-A2- 2 386 344
- WO-A1-2010/121749
- DE-U1- 29 812 154
- DE-U1-202009 004 204
- DE-U1-202012 100 997

## Beschreibung

Die vorliegende Erfindung betrifft eine medizinische Dichtung für einen medizinischen Sterilisierbehälter, welcher ein Behälterunterteil und ein Behälteroberteil zum Verschließen des Behälterunterteils umfasst, welche Dichtung in sich geschlossen ausgebildet und umlaufend am Behälteroberteil anordenbar ist, einen Dichtelementhaltekörper zum Festlegen am Behälteroberteil und zwei am Dichtelementhaltekörper angeordnete, umlaufende Dichtlippen umfasst.

Ferner betrifft die vorliegende Erfindung einen medizinischen Sterilisierbehälter mit einem wannenförmigen Behälterunterteil und einem Behälteroberteil zum Verschließen des Behälterunterteils in einer Verschlussstellung, welches Behälteroberteil eine medizinische Dichtung umfasst, welche Dichtung in sich geschlossen ausgebildet und umlaufend am Behälteroberteil angeordnet ist, einen am Behälteroberteil festgelegten Dichtelementhaltekörper und zwei am Dichtelementhaltekörper angeordnete, umlaufende Dichtlippen umfasst.

Medizinische Dichtungen und Sterilisierbehälter der eingangs beschriebenen Art sind beispielsweise aus der DE 298 12 154 U1 bekannt. Sterilisierbehälter, die auch als Sterilisationsbehälter bezeichnet werden, dienen insbesondere zur Aufnahme chirurgischer Instrumente, Implantate und dergleichen, welche in ihnen sterilisiert werden und anschließend zur Lagerung in ihnen verbleiben. Der Sterilisierbehälter muss bei der Dampfsterilisation einerseits den gesättigten Wasserdampf hineinlassen, andererseits dient er als Keimsperre bei der Lagerung. Medizinische Dichtungen für Sterilisierbehälter mit zwei Dichtlippen auszustatten hat den Vorteil, eine redundante Abdichtung eines Innenraums des Sterilisierbehälters sicherzustellen.

Eine medizinische Dichtung mit zwei Dichtlippen vorzusehen, hat jedoch auch den Nachteil, dass zum Abheben des Behälteroberteils, welches insbesondere einen Deckel für das Behälterunterteil bildet, im Vergleich zu einer Dichtung mit nur einer Dichtlippe ein zusätzlicher Kraftaufwand erforderlich ist. Der Grund ist, dass zum Abheben nicht nur die Reibung zwischen einer Dichtlippe und dem Behälterunterteil, sondern zwischen zwei Dichtlippen und dem Behälterunterteil überwunden werden muss.

Weitere medizinische Dichtungen und Sterilisierbehälter sind in der 20 2009 004 204 U1, welche den nächstliegenden Stand der Technik darstellt, offenbart. In der EP 2 386 344 A2 ist eine Dichtung für ein Gasturbinenfilter beschrieben. Die WO 2010/121749 A1 befasst sich mit Dichtstopfen.

Es ist daher eine Aufgabe der vorliegenden Erfindung, eine medizinische Dichtung sowie einen medizinischen Sterilisierbehälter der eingangs beschriebenen Art so zu verbessern, dass die Handhabung derselben vereinfacht wird.

Diese Aufgabe wird bei einer medizinischen Dichtung der eingangs beschriebenen Art erfindungsgemäß dadurch gelöst, dass mindestens eine der Dichtlippen gelenkig am Dichtelementhaltekörper gehalten oder gelagert ist.

Anders als bei elastischen Dichtlippen, die eine Beweglichkeit und lediglich aufgrund ihrer Flexibilität und Elastizität ermöglichen, hat das gelenkige Halten oder Lagern der Dichtlippe am Dichtelementhaltekörper den Vorteil, dass eine Bewegung der Dichtlippe auch als Ganzes relativ zum Dichtelementhaltekörper möglich ist. Dies gestattet es beispielsweise, die Dichtung derart anzuordnen, dass mindestens eine der Dichtlippen beim Verschließen des Sterilisierbehälters aus einer Grundstellung in eine ausgelenkte Stellung auslenkbar und insbesondere mittels herkömmlicher Verriegelungsklappen, die zum Verriegeln des Sterilisierbehälters in der Verschlussstellung insbesondere am Behälteroberteil angeordnet sind und mit entsprechenden Vorsprüngen am Behälterunterteil in der Verschlussstellung in Eingriff stehen und zusammenwirken, in der ausgelenkten Stellung gehalten werden kann. Werden beim Öffnen des Sterilisierbehälters die einen Teil eines Verriegelungsmechanismus bildenden Verriegelungsklappen wieder geöffnet oder freigegeben, kann je nach Wahl des Materials und der Art der gelenkigen Verbindung zwischen der mindestens einen Dichtlippe und dem Dichtelementhaltekörper die mindestens eine Dichtlippe insbesondere selbsttätig von der ausgelenkten Stellung in die Grundstellung zurückkehren, so dass hier aufgrund der vorgesehenen zweiten Dichtlippe trotzdem kein zusätzlicher Kraftaufwand beim Abheben des Deckels erforderlich ist, wie dies bei einem aus der DE 298 12 154 U1 bekannten Behälteroberteil der Fall ist. Die zum Abheben des Behälteroberteils vom Behälterunterteil erforderliche Abhebekraft kann so insbesondere auf einfache Weise signifikant reduziert werden. Insgesamt kann also trotz einer doppelten und damit redundanten Abdichtung des Innenraums des Sterilisierbehälters eine einfache und sichere Handhabung sichergestellt werden. Günstig ist es, dass die medizinische Dichtung einen umlaufenden Dichtlippenträger umfasst, welcher mindestens eine der Dichtlippen trägt oder umfasst und gelenkig mit dem Dichtelementhaltekörper gekoppelt oder verbunden ist. Einen solchen Dichtlippenträger vorzusehen, hat insbesondere den Vorteil, dass eine Bewegung der mindestens einen Dichtlippe relativ zum Dichtelementhaltekörper in besonders definierter Weise erfolgen kann. Gemäß der Erfindung ist ferner vorgesehen, dass der Dichtelementhaltekörper und der Dichtlippenträger über ein Gelenk miteinander gekoppelt oder verbunden sind. Vorzugsweise ist das Gelenk ein Scharniergelenk. Insbesondere kann es sich um eine Filmscharniergelenk oder filmscharnierartiges Gelenk handeln. Zum Beispiel bei einer einstückigen Ausbildung der Dichtung ist es besonders einfach, ein Gelenk, welches insbesondere filmscharnierartig geformt sein kann, auszubilden.

Besonders einfach ausbilden lässt sich die medizinische Dichtung, wenn mindestens eine der beiden Dichtlippen am Dichtelementhaltekörper verschwenkbar oder im Wesentlichen verschwenkbar gelagert ist. Eine Schwenklagerung vorzusehen gestattet es insbesondere, mindestens eine der Dichtlippen aus ihrer Grundstellung in eine ausgelenkte Stellung zu verschwenken. Dies kann beispielsweise entgegen der Wirkung einer Rückstelleinrichtung erfolgen, wobei die medizinische Dichtung die Rückstelleinrichtung umfassen kann. Denkbar ist es insbesondere, die gelenkige Verbindung zwischen der mindestens einen Dichtlippe und dem Dichtelementhaltekörper als ein von der Rückstelleinrichtung umfasstes Rückstellglied zu nutzen, um eine zunächst in der ausgelenkten Stellung gehaltene Dichtlippe ohne das Einwirken weiterer äußerer Kräfte wieder in ihre Grundstellung zurück zu überführen, wobei die in der ausgelenkten Stellung im Rückstellglied beziehungsweise in der Rückstelleinrichtung gespeicherte Kraft für die Zurücküberführung in die Grundstellung genutzt wird.

Vorteilhafterweise ist der Dichtlippenträger verschwenkbar oder im Wesentlichen verschwenkbar am Dichtelementhaltekörper gelagert. Eine solche Halterung oder Lagerung ermöglicht auf einfache Weise eine Verschwenkbewegung zwischen dem Dichtlippenträger und dem Dichtelementhaltekörper. Insbesondere kann so eine Kraft zum Verschwenken des Dichtlippenträgers vom Behälterunterteil auf eine der beiden Dichtlippen eingeleitet werden.

Günstigerweise trägt oder umfasst der Dichtlippenträger beide Dichtlippen. Dies ermöglicht es insbesondere, in Folge einer Bewegung des Dichtlippenträgers relativ zum Dichtelementhaltekörper beide Dichtlippen in definierter Weise und gleichzeitig relativ zum Dichtelementhaltekörper zu bewegen, beispielsweise zu verschieben oder zu verschwenken. Insgesamt ist so also eine gekoppelte Bewegung der beiden Dichtlippen relativ zum Dichtelementhaltekörper möglich.

Gemäß einer bevorzugten Ausführungsform der Erfindung kann vorgesehen sein, dass der Dichtlippenträger eine Innenseite und eine Außenseite umfasst und dass die beiden Dichtlippen von der Innenseite abstehend angeordnet oder ausgebildet sind. Insbesondere kann die medizinische Dichtung somit derart am Behälteroberteil angeordnet werden, dass die Innenseite des Dichtlippenträgers in Richtung auf eine nach außen weisende Seitenfläche des Behälterunterteils hin weist.

Um eine Stabilität der Dichtung zu erhöhen und insbesondere die gewünschte Dichtfunktion sicherzustellen, ist es vorteilhaft, wenn die medizinische Dichtung eine Mehrzahl von Versteifungselementen umfasst, welche jeweils mit beiden Dichtlippen verbunden oder gekoppelt sind. Die Versteifungselemente dienen insbesondere dem Zweck, eine definierte Bewegung der Dichtlippen relativ zum Dichtelementhaltekörper zu gewährleisten, und zwar aufgrund einer definierten Bewegung des Dichtlippenträgers relativ zum Dichtelementhaltekörper. Dabei ist es insbesondere denkbar, die Dichtlippen trotzdem in herkömmlicher Weise elastisch und/oder flexibel auszubilden, um eine Restelastizität beziehungsweise -flexibilität und somit optimale Anpassung an Dichtflächen des Behälterunterteils zu gewährleisten.

Eine besonders definierte Bewegung insbesondere des Dichtlippenträgers relativ zum Dichtelementhaltekörper kann erreicht werden, wenn die Mehrzahl von Versteifungselementen in Form von Versteifungsrippen ausgebildet ist, welche vorzugsweise über den Umfang des Dichtelements gleichmäßig oder im Wesentlichen gleichmäßig verteilt angeordnet sind. Günstig kann es jedoch auch sein, bei einer grundsätzlich gleichmäßig verteilten Anordnung der Versteifungselemente über den Umfang der Dichtung einzelne Versteifungselemente wegzulassen beziehungsweise gezielt nicht vorzusehen. Mit anderen Worten ist in einem solchen Bereich der Abstand zwischen benachbarten Versteifungselementen mindestens doppelt, vorzugsweise dreimal so groß wie der Abstand zwischen diesen an der Dichtung im Übrigen. So kann in einem Bereich oder Abschnitt der Dichtung mit entsprechend weit voneinander beabstandeten Versteifungselementen erreicht werden, dass die beiden Dichtlippen dann, wenn der Druck in der Umgebung des Sterilisierbehälters sehr viel größer als im Behälterinnenraum ist, aufgrund ihrer Elastizität und/oder Flexibilität ausweichen können, so dass das den Sterilisierbehälter umgebende Medium, beispielsweise Heißdampf in einem Sterilisator, in den Behälterinnenraum strömen kann. Ein solcher Bereich oder Abschnitt der Dichtung bildet somit quasi einen Druckausgleichsbereich derselben.

Um das Aufsetzen des Behälteroberteils auf ein Behälterunterteil zu erleichtern und das Behälteroberteil in definierter und zentrierter Weise auf dem Behälterunterteil in Anlage zu bringen, ist es günstig, wenn eine Mehrzahl von Zentrierelementen mit einer schräg nach außen abfallenden Aufgleitkante vorgesehen ist. Dies fördert zudem eine definierte Abdichtung des Behälterinnenraums mittels der Dichtlippen der medizinischen Dichtung.

Besonders einfach wird der Aufbau der medizinischen Dichtung, wenn die Versteifungselemente die Zentrierelemente bilden oder umfassen. Insbesondere können die Versteifungselemente somit eine Doppelfunktion ausüben. Zum einen können sie eine Stabilität der Dichtung erhöhen und zum anderen gleichzeitig eine einfache und definierte Positionierung des Behälteroberteils auf dem Behälterunterteil des Sterilisierbehälters sicherstellen.

Vorteilhaft kann es ferner sein, wenn die Dichtlippen ausgebildet sind zum Anlegen an quer oder im Wesentlichen quer zueinander verlaufende Flächen des Behälterunterteils. Vorzugsweise sind die Dichtlippen ausgebildet zum Anlegen an senkrecht zueinander verlaufende Flächen des Behälterunterteils. Beispielsweise kann die ein Dichtlippe ausgebildet sein zum Anlagen an eine in Richtung auf das Behälteroberteil weisende, umlaufende Dichtkante des Behälterunterteils, die zweite Dichtlippe zum Anlagen an eine vom Behälterunterteil weg weisende Außenfläche desselben, die beispielsweise zudem quer, insbesondere senkrecht zur ersten Dichtkante orientiert sein kann.

Günstigerweise ist eine erste Dichtlippe ausgebildet zum Anlegen an eine in der Schließstellung vom Behälterunterteil in Richtung auf das Behälteroberteil hin weisende umlaufende obere Kante. Mit dieser ersten Dichtlippe kann so eine Abdichtung des Behälteroberteils und des Behälterunterteils relativ zueinander erfolgen. So lässt sich eine Sterilitätsgrenze oder -barriere bis an den oberen Rand des Behälterunterteils hin verschieben.

Vorzugsweise ist die erste Dichtlippe am Dichtlippenträger oben angeordnet oder ausgebildet. Dies ermöglicht es, insbesondere in Folge eines in Kontakt Tretens der ersten Dichtlippe mit einer vom Behälterunterteil in Richtung auf den Behälteroberteil hin weisenden, umlaufenden Kante, die erste Dichtlippe und gegebenenfalls den Dichtlippenträger in definierter Weise relativ zum Dichtelementhalteelement zu bewegen, insbesondere zu verschwenken. Oben angeordnet oder ausgebildet kann insbesondere bedeuten, dass die erste Dichtlippe benachbart zum Dichtelementhaltekörper angeordnet oder ausgebildet ist und insbesondere in Folge einer Auslenkung aus der Grundstellung den Dichtelementhaltekörper berühren oder mit diesem in Kontakt treten kann.

Vorzugsweise ist eine zweite Dichtlippe ausgebildet zum Anlegen an eine vom Behälterunterteil weg weisende, insbesondere seitliche Außenfläche desselben. In Verbindung mit der ersten Dichtlippe ist es so beispielsweise möglich, die Sterilitätsgrenze an eine äußere Seite des Behälterunterteils zu verlegen.

Günstigerweise ist die zweite Dichtlippe am Dichtlippenträger unten angeordnet oder ausgebildet. Dies gestattet es, mit der zweiten Dichtlippe das Behälteroberteil relativ zum Behälterunterteil abzudichten unterhalb insbesondere einer vom Behälterunterteil in Richtung auf das Behälteroberteil hin weisenden Dichtkante, an welche beispielsweise die erste Dichtlippe anlegbar ist. Unten angeordnet oder ausgebildet kann insbesondere bedeuten, dass die zweite Dichtlippe vom Dichtelementhaltekörper weiter weg angeordnet oder ausgebildet ist als die oben am Dichtlippenträger angeordnete oder ausgebildete erste Dichtlippe.

Um die Steifigkeit der medizinischen Dichtung in gewünschter Weise einstellen zu können, insbesondere zu erhöhen, ist es vorteilhaft, wenn die Mehrzahl von Versteifungselementen mit dem Dichtlippenträger verbunden oder gekoppelt ist. Dadurch ist es möglich, in Folge der Einleitung einer Kraft auf eine der beiden Dichtlippen oder den Dichtlippenträger eine definierte Bewegung des Dichtlippenträgers insbesondere auch zusammen mit den beiden Dichtlippen relativ zum Dichtelementhaltekörper zu erreichen.

Günstig ist es, wenn die erste Dichtlippe über die Mehrzahl von Versteifungselementen vorstehend ausgebildet ist. So kann insbesondere erreicht werden, dass die erste Dichtlippe mit einem Dichtlippenabschnitt, welcher über die Versteifungselemente vorsteht, an eine Dichtkante oder Dichtfläche des Behälterunterteils in Anlage gebracht werden kann, wobei eine Elastizität oder Flexibilität der Dichtlippe in diesem Bereich genutzt werden kann, um eine sichere Abdichtung des Behälterinnenraums zu erreichen.

Gemäß einer weiteren bevorzugten Ausführungsform der Erfindung kann vorgesehen sein, dass die erste Dichtlippe in einer Grundstellung des Dichtelements, in welcher die beiden Dichtlippen relativ zum Dichtelementhaltekörper unausgelenkt sind, schräg nach unten vom Dichtelementhaltekörper weg geneigt ist. Mit anderen Worten wird so zwischen dem Dichtelementhaltekörper und der ersten Dichtlippe ein Öffnungswinkel definiert, welcher in einem Bereich vorzugsweise zwischen 0 und 45° liegt, insbesondere in einem Bereich von etwa 10° bis 35°. Ein solcher Öffnungswinkel ermöglicht insbesondere eine Schwenkbewegung der ersten Dichtlippe, beispielsweise auch zusammen mit dem Dichtelementträger, in Richtung auf den Dichtelementhaltekörper hin um einen dem Öffnungswinkel entsprechenden Winkel beim Übergang von der Grundstellung des Dichtelements in eine ausgelenkte Stellung.

Vorteilhaft ist es, wenn ein zwischen der ersten Dichtlippe und dem Dichtlippenträger eingeschlossener Winkel in der Grundstellung in einem Bereich von etwa 45° bis etwa 85° liegt. Insbesondere wenn der Dichtlippenträger in einer Grundstellung des Dichtelements senkrecht oder im Wesentlichen senkrecht vom Dichtelementhaltekörper abstehend ausgebildet ist, ermöglicht der Winkel zwischen der ersten Dichtlippe und dem Dichtelementträger im angegebenen Bereich eine Verschwenkbewegung des Dichtlippenträgers um einen Winkel in einem Bereich von 5° bis 45° auf den Dichtelementhaltekörper hin. Insbesondere kann die Auslenkung in elastischer Weise erfolgen, so dass das Dichtelement selbsttätig in die Grundstellung zurückgeht, wenn keine Kraft mehr auf den Dichtlippenträger oder insbesondere die erste Dichtlippe ausgeübt wird, um den Sterilisierbehälter in der Schließstellung abgedichtet zu halten.

Günstig kann es ferner sein, wenn die zweite Dichtlippe in einer Grundstellung, in welcher die beiden Dichtlippen relativ zum Dichtelementhaltekörper unausgelenkt sind, schräg nach oben auf den Dichtelementhaltekörper hin geneigt ist. Eine solche Ausgestaltung der zweiten Dichtlippe ermöglicht insbesondere ein im Wesentlichen kräftefreies Aufsetzen des Behälteroberteils auf das Behälterunterteil. Ferner kann sich die zweite Dichtlippe so insbesondere mit einer von der ersten Dichtlippe weg weisenden Außenfläche an eine Außenseite des Behälterunterteils anschmiegen.

Günstigerweise liegt ein zwischen der zweiten Dichtlippe und dem Dichtlippenträger eingeschlossener zweiter Winkel in der Grundstellung in einem Bereich von etwa 70° bis etwa 90°. Insbesondere dann, wenn der Dichtlippenträger in der Grundstellung senkrecht vom Dichtelementhaltekörper abstehend ausgebildet ist, kann so eine leicht schräg in Richtung auf die erste Dichtlippe hin ausgerichtete zweite Dichtlippe realisiert werden.

Vorteilhafterweise ist die erste Dichtlippe länger als die zweite Dichtlippe. Vorzugsweise ist die erste Dichtlippe mindestens etwa doppelt so lang wie die zweite Dichtlippe. Damit kann die erste Dichtlippe beispielsweise eine obere Kante des Behälterunterteils übergreifen und an diese insbesondere in der Schließstellung in Anlage gebracht werden. Die zweite Dichtlippe, die vorzugsweise eine vom Behälterunterteil weg weisende Außenseite desselben abdichtet, wird vorzugsweise kürzer und nur so lang ausgebildet, dass eine sichere Abdichtung gewährleistet ist. Damit können insbesondere Kräfte beim Verschließen und Öffnen des Sterilisierbehälters optimiert werden, also beispielsweise beim Abheben des Behälteroberteils vom Behälterunterteil minimiert.

Um insbesondere eine Verschwenkbewegung des Dichtlippenträgers relativ zum Dichtelementhaltekörper zu verbessern, ist die erste Dichtlippe günstigerweise dicker als die zweite Dichtlippe ausgebildet. Vorzugsweise ist die erste Dichtlippe mindestens etwa doppelt so dick wie die zweite Dichtlippe. Damit kann insbesondere eine beim Aufsetzen des Behälteroberteils von einer auf das Behälteroberteil hin weisenden Kante des Behälterunterteils wirkende Kraft auf die erste Dichtlippe genutzt werden, um diese in Richtung auf den Dichtelementhaltekörper hin und damit auch den Dichtlippenträger synchron zu verschwenken, was es insbesondere gestattet, die zweite Dichtlippe erst in Folge einer solchen Verschwenkbewegung mit einer Außenfläche des Behälterunterteils in Kontakt zu bringen. Umgekehrt kann beim Abheben des Behälteroberteils die aufgrund der Auslenkung des Dichtlippenträgers relativ zum Dichtelementhaltekörper gespeicherte Kraft genutzt werden, um ein Abheben des Behälteroberteils vom Behälterunterteil zu forcieren. Die gespeicherte Kraft beziehungsweise Energie kann also zum einfacheren Abheben des Behälteroberteils vom Behälterunterteil genutzt werden.

Vorteilhaft ist es, wenn der Dichtlippenträger auf seiner Außenseite eine Mehrzahl von Wandabstandshalteelementen umfasst. Die Wandabstandshalteelemente stellen insbesondere sicher, dass der Dichtlippenträger von einer umlaufenden Wandfläche des Behälteroberteils in der Grundstellung in einem definierten Abstand gehalten wird. Dies verbessert insbesondere eine Hinterspülung der Dichtung beim Reinigen. Ferner kann so insbesondere auch eine Steifigkeit der Dichtung zusätzlich erhöht werden.

Besonders einfach ausbilden lässt sich die medizinische Dichtung, wenn die Mehrzahl von Wandabstandshalteelementen in Form von Vorsprüngen ausgebildet ist. Vorteilhafterweise sind die Wandabstandshalteelemente in Form von rippenförmigen Vorsprüngen ausgebildet. So kann insbesondere eine Anlagefläche des Dichtlippenträgers an einer Wandfläche des Behälteroberteils minimiert und eine optimale Hinterspülung der Dichtung sowie die erforderliche Steifigkeit der Dichtung erreicht werden.

Günstig ist es, wenn die Dichtung ein Anschlagselement umfasst, an welchem die erste Dichtlippe in einer Dichtstellung, in welcher die Dichtlippen relativ zum Dichtelementhaltekörper aus der Grundstellung ausgelenkt sind, anschlägt. Insbesondere ist es für einen einfachen Aufbau der Dichtung günstig, wenn der Dichtelementhaltekörper das Anschlagelement bildet oder umfasst.

Vorzugsweise ist das Anschlagelement in Form eines im Querschnitt dreieckigen oder im Wesentlichen dreieckigen, umlaufenden Profils ausgebildet. Ein solches Profil ist einfach und kostengünstig herzustellen.

Vorteilhaft ist es, wenn die erste Dichtlippe mit einer Mehrzahl von Aussparungen versehen ist. Vorzugsweise sind die Aussparungen ausgehend von einer vorderen Dichtlippenkante in Form von im Wesentlichen halbkreisförmigen Einschnitten ausgebildet. Die Aussparungen verhindern insbesondere, dass es beim Aufsetzen des Behälteroberteils auf das Behälterunterteil zu einer Faltenbildung kommen kann, welche eine sichere Abdichtung des Behälterinnenraums des Sterilisierbehälters in Frage stellen könnte.

Vorzugsweise ist die medizinische Dichtung einstückig ausgebildet. So kann auf einfache und sichere Weise eine umlaufende Abdichtung des Behälteroberteils und des Behälterunterteils relativ zueinander insbesondere in der Schließstellung sichergestellt werden.

Vorteilhaft ist es, wenn die medizinische Dichtung eine Mehrzahl von Befestigungselementaufnahmen zum Aufnehmen jeweils eines Befestigungselements zum Festlegen des Dichtelements am Behälteroberteil umfasst. Vorzugsweise sind die Befestigungselementaufnahmen in Form von Durchbrechungen des Dichtelementhaltekörpers ausgebildet. Beispielsweise ist es so möglich, am Behälteroberteil angeordnete Haltezapfen oder anderweitig ausgebildete Befestigungselemente in korrespondierend angeordneten Befestigungselementaufnahmen aufzunehmen. Vorzugsweise sind die Befestigungselementaufnahmen mit Hinterschneidungen versehen, beispielsweise in Form von ringförmigen Hinterschneidungen, in welche ringförmige Vorsprünge an den Befestigungselementen eingreifen können, um einen sicheren Halt des Dichtelementhaltekörpers am Behälteroberteil sicherzustellen.

Günstig ist es, wenn am Dichtelementhaltekörper und/oder am Dichtlippenträger auf von den Dichtlippen weg weisenden Außenflächen Ausnehmungen ausgebildet sind zur Ausbildung eines Überdruckströmungskanals. Eine solche Ausgestaltung ermöglicht es insbesondere, dass der Dichtlippenträger beim Verschließen des Sterilisierbehälters einen durch die Ausnehmungen und der Wand des Behälteroberteils definierten Überdruckströmungskanal freigibt, so dass im Falle eines gegenüber einem in der Umgebung des Sterilisierbehälters herrschenden Drucks im Behälterinnenraum herrschenden Unterdrucks ein von den Dichtlippen weg weisendes Ende des Dichtelementhaltekörpers von der Wand des Behälteroberteils abheben kann, so dass über den dann beidseits geöffneten Überdruckströmungskanal ein Druckausgleich zwischen dem Behälterinnenraum und der Umgebung ermöglicht wird.

Die eingangs gestellte Aufgabe wird ferner bei einem medizinischen Sterilisierbehälter der eingangs beschriebenen Art erfindungsgemäß dadurch gelöst, dass mindestens eine der Dichtlippen gelenkig am Dichtelementhaltekörper gehalten oder gelagert ist.

Wie bereits oben näher erläutert, kann eine derart angeordnete und/oder ausgebildete Dichtlippe genutzt werden, um eine in Folge einer Auslenkung derselben in der Dichtung gespeicherte Kraft zu einem leichteren Abheben des Behälteroberteils vom Behälterunterteil nach dessen Freigabe zu nutzen.

Vorteilhafterweise umfasst der medizinische Sterilisierbehälter eine der oben beschriebenen medizinischen Dichtungen. Der Sterilisierbehälter weist dann die oben im Zusammenhang mit bevorzugten Ausführungsformen beschriebenen Vorteile auf.

Günstig ist es, wenn an der Dichtung mindestens ein Überdruckströmungskanal ausgebildet ist, welcher in der Grundstellung der Dichtung geschlossen und in der Schließstellung zur Umgebung des Sterilisierbehälters hin geöffnet ist. Eine solche Ausgestaltung ermöglicht es insbesondere, dass der Dichtlippenträger beim Verschließen des Sterilisierbehälters einen durch den Überdruckströmungskanal definierten Belüftungskanal freigibt, so dass im Falle eines gegenüber einem in der Umgebung des Sterilisierbehälters herrschenden Drucks im Behälterinnenraum herrschenden Unterdrucks ein von den Dichtlippen weg weisendes Ende des Dichtelementhaltekörpers von einer Wand des Behälteroberteils abheben kann, so dass über den dann beidseits geöffneten Überdruckströmungskanal ein Druckausgleich zwischen dem Behälterinnenraum und der Umgebung ermöglicht wird.

Die nachfolgende Beschreibung bevorzugter Ausführungsformen der Erfindung dient im Zusammenhang mit der Zeichnung der näheren Erläuterung. Es zeigen:
- Figur 1:: eine teilweise durchbrochene, schematische Gesamtansicht eines erfindungsgemäßen Sterilisierbehälters;

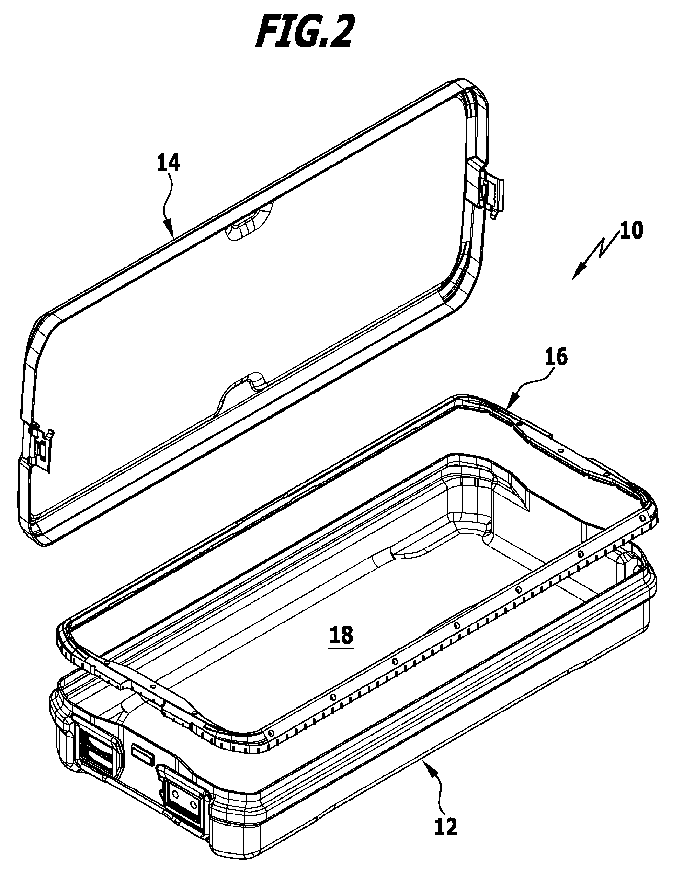

- Figur 2:: eine Darstellung des Sterilisierbehälters aus Figur 1 mit abgehobenem Behälteroberteil und von diesem getrennter medizinischer Dichtung;

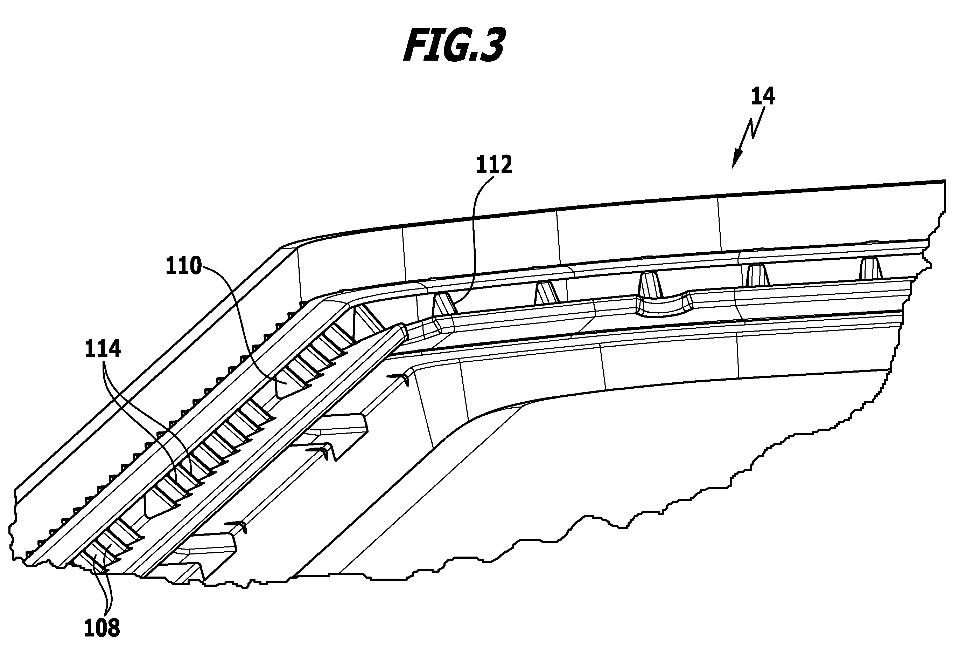

- Figur 3:: eine Teilausschnittsansicht eines Eckbereichs des Behälteroberteils mit eingesetzter Dichtung;

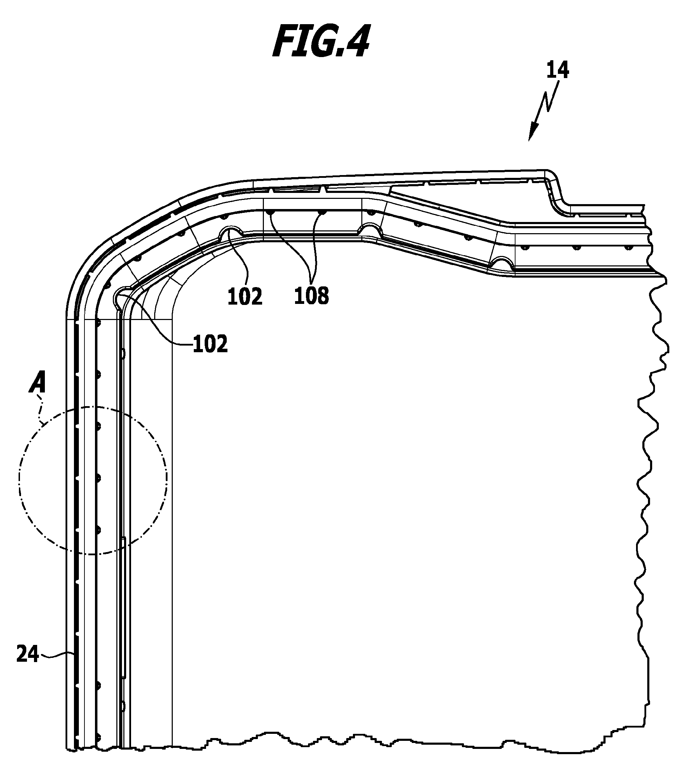

- Figur 4:: eine teilweise Draufsicht auf das Behälteroberteil von unten;

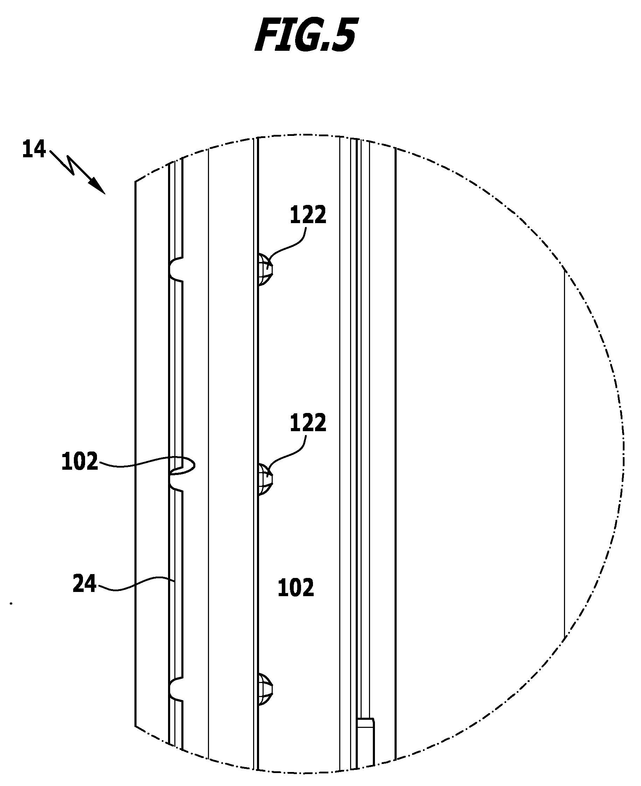

- Figur 5:: eine vergrößerte Ansicht des Bereichs A in Figur 4;
- Figur 6:: eine weitere Ansicht des Behälteroberteils von unten ähnlich Figur 3;
- Figur 7:: eine ausschnittsweise Schnittansicht des Sterilisierbehälters vor dem Aufsetzen des Behälteroberteils auf das Behälterunterteil;
- Figur 8:: eine Ansicht ähnlich Figur 7 nach Aufsetzen des Behälteroberteils auf das Behälterunterteil in der Schließstellung;
- Figur 9:: eine geschnittene Ansicht eines Abschnitts der medizinischen Dichtung; und
- Figur 10:: eine Ansicht der Dichtung ähnlich Figur 9 von der Gegenseite;
- Figur 11A:: eine ausschnittsweise Schnittansicht des Sterilisierbehälters vor dem Aufsetzen des Behälteroberteils auf das Behälterunterteil mit einer alternativen Ausführungsform einer medizinischen Dichtung;
- Figur 11B:: eine Ansicht ähnlich Figur 11A nach dem Aufsetzen des Behälterteils auf das Behälterunterteil in der Schließstellung;
- Figur 11C:: eine Ansicht ähnlich Figur 11B, jedoch bei einem in der Umgebung des Sterilisierbehälters herrschenden Druck, welcher den im Behälterinnenraum herrschenden Druck signifikant übersteigt; und
- Figur 11D:: eine ausschnittsweise Schnittansicht des Sterilisierbehälters vor dem Aufsetzen des Behälteroberteils auf das Behälterunterteil ähnlich Figur 11A, jedoch im Bereich eines Befestigungselements.

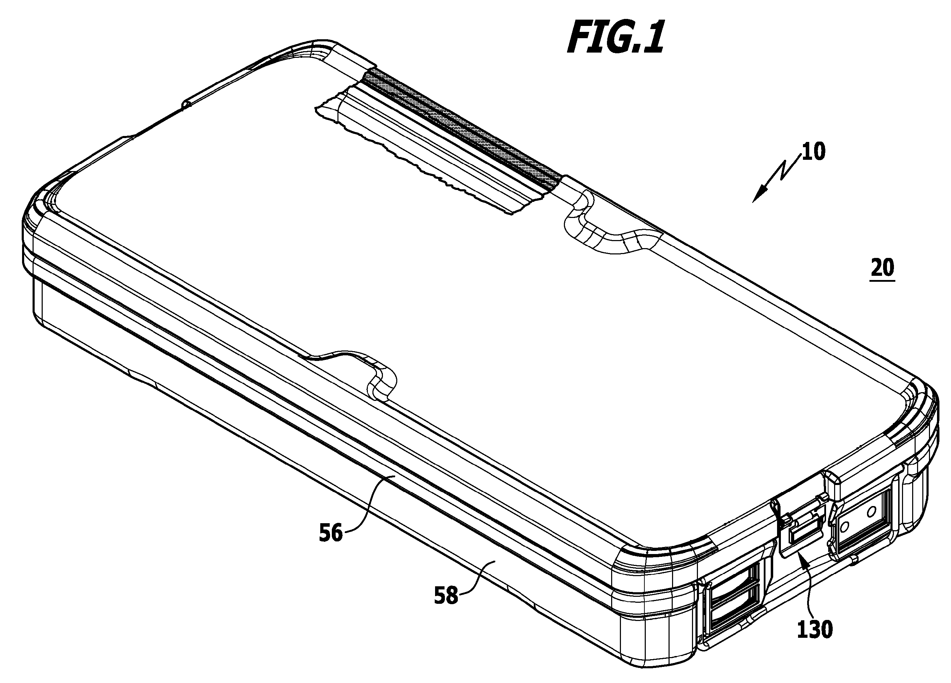

In Figur ist schematisch ein erstes Ausführungsbeispiel eines medizinischen Sterilisierbehälters dargestellt und insgesamt mit dem Bezugszeichen 10 bezeichnet. Dieser umfasst ein Behälterunterteil 12 in Form einer Wanne und ein Behälteroberteil 14 in Form eines Deckels zum Verschließen des Behälterunterteils 12 in einer in Figur 1 dargestellten Verschlussstellung. Am Behälteroberteil 12 ist eine medizinische Dichtung 16 angeordnet, um einen vom Sterilisierbehälter 10 definierten Behälterinnenraum 18 gegenüber einer Umgebung 20 des Sterilisierbehälters 10 abzudichten.

Die Dichtung 16 ist vorzugsweise einstückig ausgebildet und umfasst einen Dichtelementhaltekörper 22 sowie eine erste Dichtlippe 24 und eine zweite Dichtlippe 26. Der Dichtelementhaltekörper 22 ist in Form eines umlaufenden, strangförmigen, im Querschnitt dreieckigen oder im Wesentlichen dreieckigen Profils 28 ausgebildet. Die beiden Dichtlippen 24 und 26 sind an einem umlaufenden, vorzugsweise flachbandartigen Dichtlippenträger 30 angeordnet beziehungsweise ausgebildet, und zwar auf dessen in Richtung auf den Behälterinnenraum 18 hin weisenden Innenseite 134. Der Dichtlippenträger 30 und damit auch die Dichtlippen 24 und 26 sind gelenkig mit dem Dichtelementhaltekörper 22 verbunden. Ein Gelenk 32 wird ausgebildet durch einen schmalen Steg 34 zwischen dem Dichtelementhaltekörper 22 und dem Dichtlippenträger 30. Insgesamt entsteht so eine Art Scharniergelenk 36, über welches der Dichtelementhaltekörper 22 und der Dichtlippenträger 30 miteinander gekoppelt beziehungsweise verbunden sind und welches eine Verschwenkbewegung des Dichtlippenträgers 30 relativ zum Dichtelementhaltekörper 22 ermöglicht.

Der Dichtelementhaltekörper 22 weist zwei etwa um 45° gegeneinander geneigte Flächenbereiche 38 und 40 auf, welche bei bestimmungsgemäßer Anordnung der Dichtung 16 am Behälteroberteil 14 an entsprechenden Flächenbereichen 42 und 44 desselben flächig anliegen. Der Flächenbereich 44 bildet eine Innenseite einer Wand 46 des Behälteroberteils 14 und weist in Richtung auf das Behälterunterteil 12 hin. Der Flächenbereich 42 bildet eine Innenseite eines gegenüber der Wand 46 um etwa 45° geneigten Wandabschnitts 48, von welchem ein nochmals um etwa 45° geneigter Wandabschnitt 50 absteht, welcher somit im Wesentlichen senkrecht zur Wand 46 orientiert ist und einen umlaufenden Rand 52 des Behälteroberteils 14 bildet. Der Rand 52 weist in der Schließstellung in Richtung auf eine nach außen vorspringende Sicke 56 in einer umlaufenden Wand 58 des Behälterunterteils 12 hin.

Der Rand 52 umgibt in der Schließstellung einen oberen Randbereich 60 des Behälterunterteils 12 im Wesentlichen mit konstantem Abstand 62 zwischen einer Innenseite 64 des Wandabschnitts 50 und einer Außenseite 66 des Randbereichs 60. Eine Stirnkante 68 des Randbereichs 60 weist in Richtung auf das Behälteroberteil 14 hin und verläuft in etwa parallel zum Flächenbereich 44.

Der Dichtelementhaltekörper 22 ist mit einer Vielzahl von Befestigungselementaufnahmen 70 zum Aufnehmen jeweils eines vom Behälteroberteil 14 abstehenden Befestigungselements 72 zur Festlegung der Dichtung 16 am Behälteroberteil 12 versehen. Wie schematisch in den Figuren dargestellt, können die Befestigungselementaufnahmen 70 insbesondere in Form von Durchbrechungen 74 ausgebildet sein.

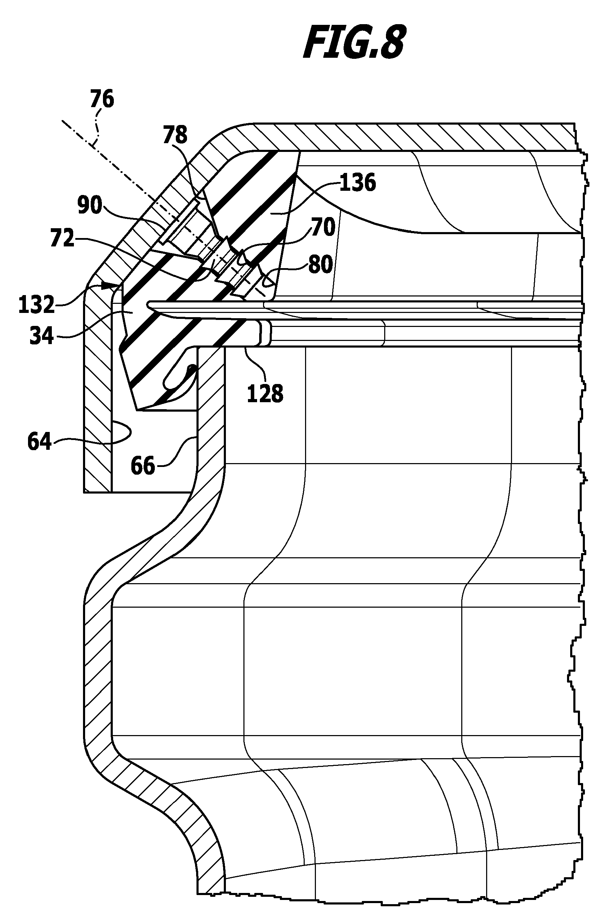

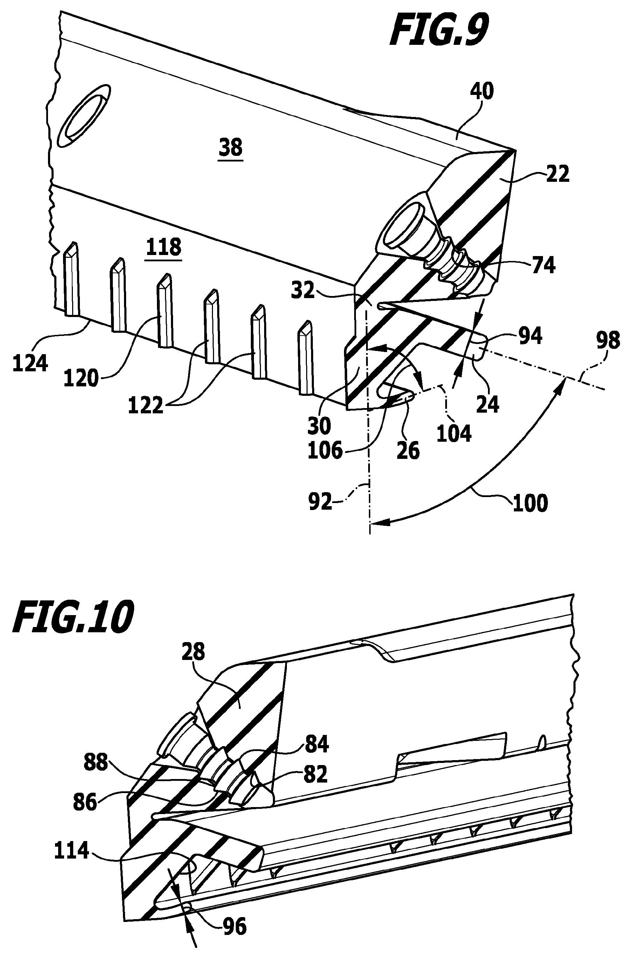

Die Befestigungselementaufnahmen 70 sind vorzugsweise rotationssymmetrisch bezogen auf eine senkrecht zum Flächenbereich 42 orientierte Längsachse 76 ausgebildet. Sie umfassen einen sich konisch in Richtung auf den Flächenbereich 42 hin erweiternden Einführabschnitt 78 sowie einen sich daran anschließenden zylindrischen Abschnitt 80, welcher mit mehreren, in den Figuren sind schematisch drei dargestellt, Rücksprüngen 82 versehen ist, welche mit korrespondierenden ringförmigen Vorsprüngen 84 am Befestigungselement 72 zusammenwirken, um eine Bewegung des Dichtelementhaltekörpers 22 vom Behälteroberteil 14 weg zu verhindern. Die Vorsprünge 84 weisen zu diesem Zweck von der Wand 48 weg weisend geneigte Ringflächen 86 auf sowie parallel zum Flächenbereich 42 verlaufende Ringflächen 88. Die dübelartigen Befestigungselemente 72 verhaken sich dann wie schematisch in den Figuren 8 bis 10 dargestellt in den Rücksprüngen 82 und verhindern eine Bewegung des Dichtelementhaltekörpers 22 vom Wandabschnitt 48 weg in Richtung auf den Behälterinnenraum 18 hin.

Für eine stabile Montage der Dichtung 16 am Behälteroberteil 14 ist eine Mehrzahl von in gleichmäßigem Abstand über den Umfang verteilt angeordneten Befestigungselementen 72 am Wandabschnitt 48 vorgesehen, welche beispielsweise durch Nieten, Schweißen oder Löten befestigt sind. Auch das Ankleben einer ebenen Endfläche 90 eines Befestigungselements 72 am Flächenbereich 42 ist denkbar. Zur Montage der Dichtung 16 wird dann lediglich der Dichtelementhalterkörper 22 so positioniert, dass die Befestigungselemente 72 in die Befestigungselementaufnahmen 70 eingeführt werden können und der Dichtelementhaltekörper 22 dann ohne weitere Hilfsmittel am Behälteroberteil 14 halten.

Der Dichtlippenträger definiert eine Hauptachse 92, die parallel zum Wandabschnitt 50 verläuft und somit in einer Grundstellung der Dichtung 16 im Wesentlichen senkrecht zum Flächenbereich 40 orientiert ist. Die erste Dichtlippe 24 weist eine Dicke 94 auf, welche etwa drei Mal so groß ist wie eine Dicke 96 der zweiten Dichtlippe. Vorzugsweise ist die erste Dichtlippe mindestens etwa doppelt so dick wie die zweite Dichtlippe 26. Die erste Dichtlippe 24 definiert eine Hauptachse 98, welche mit der Hauptachse 92 einen Winkel 100 einschließt, welcher vorzugsweise in einem Bereich von etwa 45° bis etwa 85° liegt.

Die zweite Dichtlippe 26 definiert eine Hauptachse 104, welche mit der Hauptachse 92 einen Winkel 106 einschließt, welcher in einer Grundstellung der Dichtung 16 vorzugsweise in einem Bereich von etwa 70° bis etwa 90° liegt. Somit weist die zweite Dichtlippe 26 in der Grundstellung, in welcher die beiden Dichtlippen 24 und 26 relativ zum Dichtelementhaltekörper 22 unausgelenkt sind, etwas schräg nach oben auf den Dichtelementhaltekörper 22 hin. Die erste Dichtlippe 24 weist in der Grundstellung der Dichtung 16 etwas schräg nach unten vom Dichtelementhaltekörper 22 weg.

In Eckbereichen oder Bereichen, in denen die Dichtung 16 nicht geradlinig verläuft, sondern gekrümmt oder gebogen ist, sind Aussparungen 102 vorgesehen, welche verhindern, dass es beim Aufsetzen des Behälteroberteils 14 mit der Dichtung 16 auf der Stirnkante 68 zu einer Faltenbildung der ersten Dichtlippe 24 kommt. Die Aussparungen sind ausgehend von einer vorderen Kante 138 der ersten Dichtlippe 24 in Form von halbkreisförmigen Einschnitten ausgebildet.

Zwischen den beiden Dichtlippen 24 und 26 sind gleichmäßig über den Umfang verteilt Versteifungselemente 108 angeordnet, welche in Form von im Wesentlichen dreieckigen Versteifungsrippen 110 ausgebildet sind, die die beiden Dichtlippen 24 und 26 miteinander sowie mit dem Dichtlippenträger 30 koppeln. Sie geben der Dichtung 16 die notwendige Stabilität und verhindern im Wesentlichen eine Verformung der Dichtlippen 24 und 26 unabhängig voneinander. Die erste Dichtlippe 24 ist über die Versteifungselemente 108 vorstehend ausgebildet. Die Versteifungselemente 108 bilden gleichzeitig Zentrierelemente 112 mit einer schräg in Richtung auf den Behälterinnenraum 18 hin weisenden Aufgleitkante 114. Die Zentrierelemente 112 dienen als Zentrierhilfe beim Aufsetzen des Behälteroberteils 14 auf das Behälterunterteil 12, um das Behälteroberteil 14 definiert zu positionieren. Insbesondere kann so auf einfache Weise sichergestellt werden, dass der Abstand 62 im Wesentlichen konstant ist, und zwar unabhängig davon, wo er am Sterilisierbehälter 10 gemessen wird.

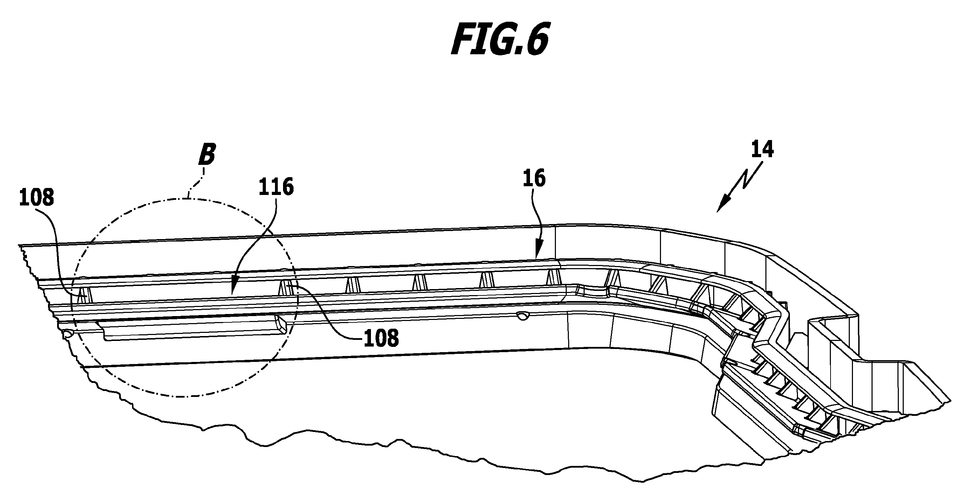

Um zumindest abschnittsweise eine Bewegung der Dichtlippen 24 und 26 unabhängig voneinander zu ermöglichen, sind verschiedene Druckausgleichsbereiche vorgesehen, wie beispielsweise in Figur 6 schematisch durch den gestrichelten Kreis B angedeutet. In den Druckausgleichsbereichen 116 fehlen praktisch zwei Versteifungselemente 108, so dass der Abstand zwischen den beiden, jeweils einen Druckausgleichsbereich 116 begrenzenden Versteifungselementen 108 etwa drei Mal so groß ist wie zwischen den Versteifungselementen 108, die im Übrigen benachbart zueinander angeordnet sind.

Ferner sind auf der der Innenseite 64 zugewandten Außenseite 118 des Dichtlippenträgers 30 eine Mehrzahl äquidistant angeordneter und parallel zueinander verlaufender Wandabstandshalteelemente 120 angeordnet. Diese sind in Form von rippenförmigen Vorsprüngen 122 ausgebildet, die sich ausgehend von einer vom Dichtelementhaltekörper 22 weg weisenden Kante 124 des Dichtlippenträgers 30 über etwa zwei Drittel einer Höhe, gemessen von der Kante 124 bis zum Gelenk 32, desselben erstrecken. Sie liegen in einer Grundstellung der Dichtung 16 an der Innenseite 64 an und halten somit den Dichtlippenträger 30 in einem definierten Abstand vom Rand 52. Dies ermöglicht eine optimale Hinterspülung des Dichtlippenträgers 30 beim Reinigen und dient der zusätzlichen Erhöhung der Steifigkeit der Dichtung 16, und zwar insbesondere im Bereich des Dichtlippenträgers 30.

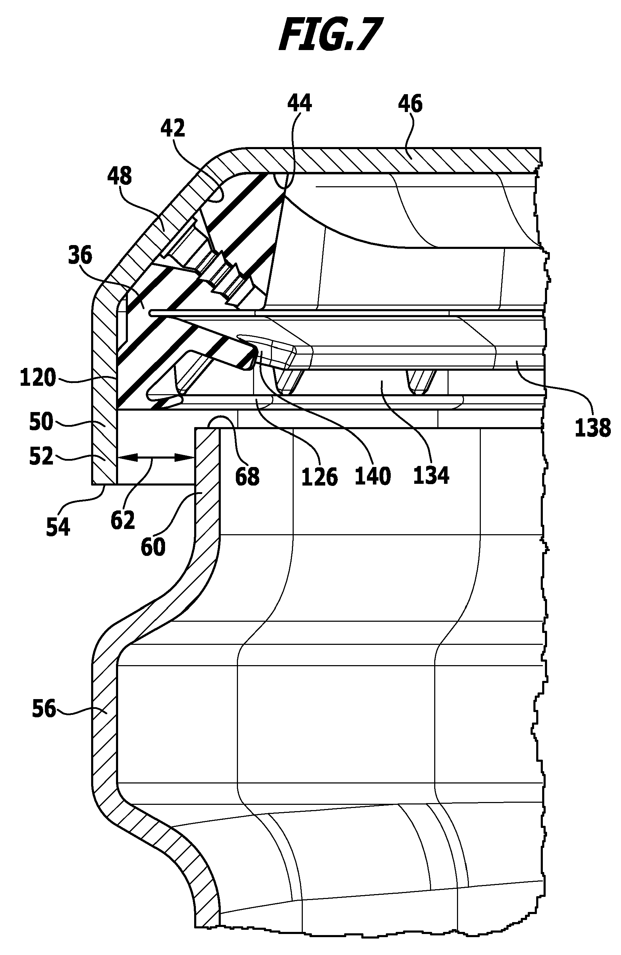

Die Funktionsweise der Dichtung 16 wird nachfolgend insbesondere in Verbindung mit den Figuren 7 und 8 näher erläutert.

Wie in Figur 7 schematisch dargestellt, liegen die Wandabstandshalteelemente 120 an der Innenseite 64 an. Eine vordere Kante 126 der zweiten Dichtlippe 26 reicht nicht bis an die Außenseite 66 heran. Wird das Behälteroberteil 14 in Richtung auf das Behälterunterteil 12 hin geführt, gleitet zunächst die Stirnkante 68 an den Aufgleitkanten 114 auf, bis sie mit der ersten Dichtlippe 24 in Kontakt tritt. Aufgrund der Kopplung der Dichtlippen 24 und 26 miteinander über die Versteifungselemente 108, führt eine weitere Bewegung des Behälteroberteils 14 in Richtung auf das Behälterunterteil 12 hin dazu, dass die von der Stirnkante 68 auf die erste Dichtlippe 24 eingeleitete Kraft zu einer Verschwenkbewegung führt, die durch das Scharniergelenk 36 definiert geführt wird. Im Extremfall kann die erste Dichtlippe 24 an den Dichtelementhaltekörper 22 anschlagen, so dass dieser ein Anschlagelement 136 bildet. Das Verschwenken der ersten Dichtlippe 24 in Richtung auf den Dichtelementhaltekörper 22 hin bewirkt dann jedoch, dass der Dichtlippenträger 30 vom Rand 52 in Richtung auf den Randbereich 60 hin verschwenkt wird. Dabei tritt dann auch die zweite Dichtlippe 26 in Kontakt mit der Außenseite 66. In der in Figur 8 dargestellten Schließstellung liegt somit die auf die zweite Dichtlippe 26 hin weisende Seitenfläche 128 der ersten Dichtlippe 24 an der Stirnkante 68 an und bildet eine erste Sterilbarriere. Die zweite Dichtlippe 26 bildet eine zweite Sterilbarriere etwas unterhalb der Stirnkante 68 auf der Außenseite 66. Somit wird praktisch eine doppelte Dichtung ausgebildet, welche eine redundante Abdichtung des Behälterinnenraums 18 ermöglicht.

Um den Sterilisierbehälter 10 in der in Figur 8 dargestellten Verschlussstellung zu halten, dienen zwei an Stirnseiten des Behälteroberteils 14 in herkömmlicher Weise verschwenkbar gelagerte Verschlussklappen 130, die mit korrespondierenden, nicht näher dargestellten Elementen, beispielsweise Vorsprüngen, welche am Behälterunterteil 12 angeordnet oder ausgebildet sind, in Eingriff bringbar sind, um das Behälteroberteil 14 in der Schließstellung am Behälterunterteil 12 zu halten.

Zum Öffnen des Sterilisierbehälters 10 werden die Verschlussklappen 130 vom Behälterunterteil 12 weg verschwenkt, so dass das Behälteroberteil 14 wieder vom Behälterunterteil 12 abgehoben werden kann. Werden die Verschlusskappen 130 geöffnet, wirkt bereits die Dichtung 16, die vorzugsweise aus einem elastischen und flexiblen Kunststoff ausgebildet ist, gleichzeitig als eine Art Rückstelleinrichtung 132, um die aus der Grundstellung ausgelenkte Dichtung 16, insbesondere den aus der Grundstellung ausgelenkten Dichtlippenträger 30, wieder in die Grundstellung zurück zu verschwenken. Die in der ausgelenkten Schließstellung in der Dichtung 16 gespeicherte Kraft unterstützt das Abheben des Behälteroberteils 14, indem die erste Dichtlippe 24 eine Kraft in Richtung der Stirnkante 68 ausübt und das Vergrößern des Abstands zwischen der ersten Dichtlippe 24 und dem Dichtelementhaltekörper 22 forciert. Dies führt gleichzeitig dazu, dass in Folge einer Verschwenkbewegung der ersten Dichtlippe 24 und damit auch des Dichtlippenträgers 30 und der an dieser angeordneten zweiten Dichtlippe 26, die zweite Dichtlippe 26 von der Außenseite 66 wieder weg verschwenkt wird, bis diese die Außenseite komplett freigibt, so dass keine weiteren Reibungskräfte überwunden werden müssen, um das Behälteroberteil 14 vom Behälterunterteil 12 abzuheben.

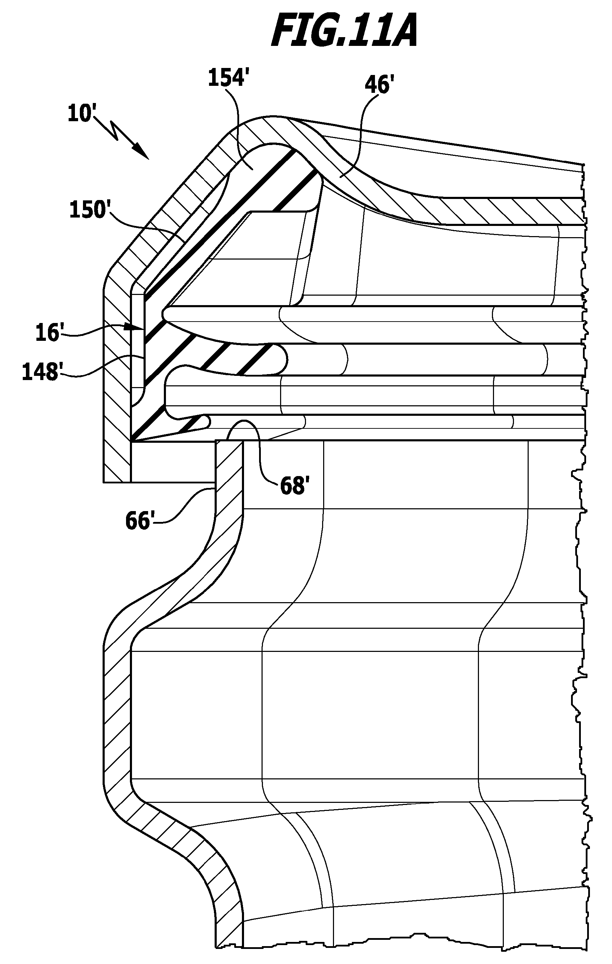

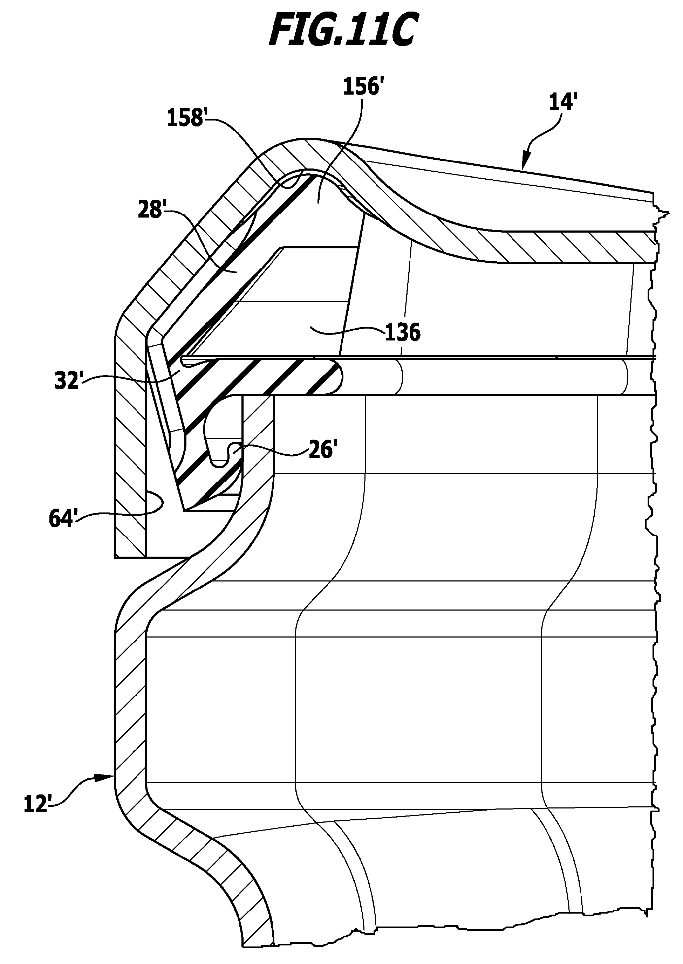

In den Figuren 11A bis 11D ist ein weiteres Ausführungsbeispiel eines Sterilisierbehälters ausschnittsweise dargestellt und insgesamt mit dem Bezugszeichen 10' bezeichnet. Teile des Sterilisierbehälters 10', welche Teilen des Sterilisierbehälters 10 entsprechen oder identisch mit diesen ausgebildet sind, sind mit denselben Bezugszeichen mit zusätzlichem "'" bezeichnet.

Der Sterilisierbehälter 10' umfasst eine Dichtung 16', welche in ihrem grundsätzlichen Aufbau der Dichtung 16 entspricht, so dass nachfolgend lediglich die Unterschiede in deren Konstruktion näher erläutert werden.

Anders als bei der Dichtung 16 sind die Befestigungselementaufnahmen 70' nicht in Form von Durchbrechungen, sondern in Form von rotationssymmetrischen Ausnehmungen 142', die auch als Sacklöcher 143' bezeichnet werden können. Die Befestigungselemente 72' sind in Form von rotationssymmetrisch ausgebildeten Zapfen ausgebildet, welche eine Ringnut 144' aufweisen, welche eine Hinterschneidung bildet, in welche ein in Richtung auf die Längsachse 76' hin vorspringender Ringvorsprung 146' im Bereich des Sacklochs 144' eingreift, wenn die Dichtung 16' am Behälteroberteil 14' gehalten ist.

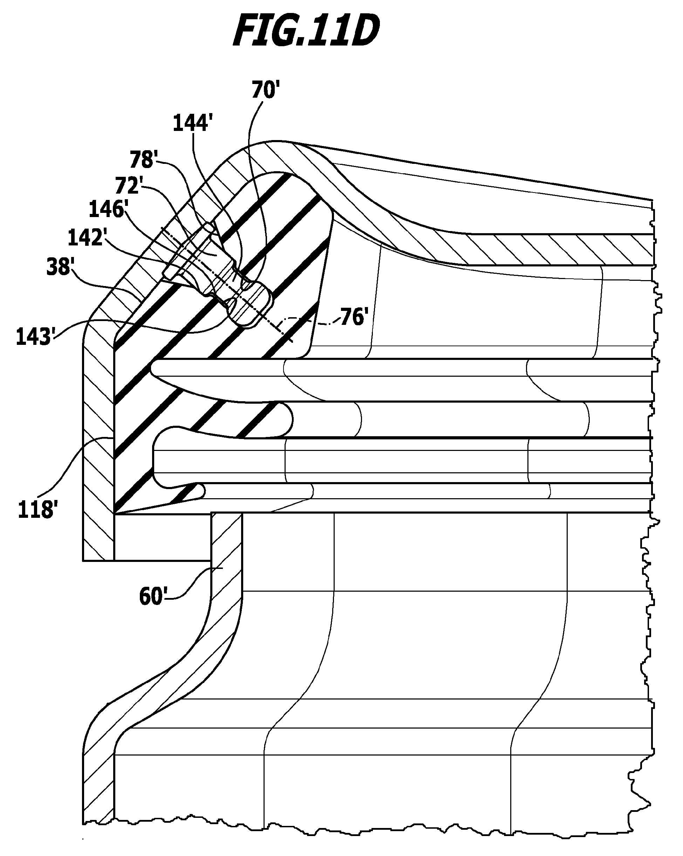

Ferner weist die Dichtung 16' keine Wandabstandshalteelemente auf. Vielmehr sind in der Außenseite 118' des Dichtlippenträgers 30' flache streifenförmige Ausnehmungen 148' ausgebildet. Diese stehen jeweils mit eine ebenfalls flachen, streifenförmigen Ausnehmung 150', welche am Flächenbereich 38' ausgebildet ist, in Fluidverbindung und bilden zusammen eine Mehrzahl von Überdruckströmungskanälen 152 aus. In der in den Figuren 11A und 11D dargestellten Grundstellung der Dichtung 16' bilden die Überdruckströmungskanäle 152 geschlossene Hohlräume aus, die einerseits von der Dichtung 16' und andererseits vom Behälteroberteil 14' begrenzt werden.

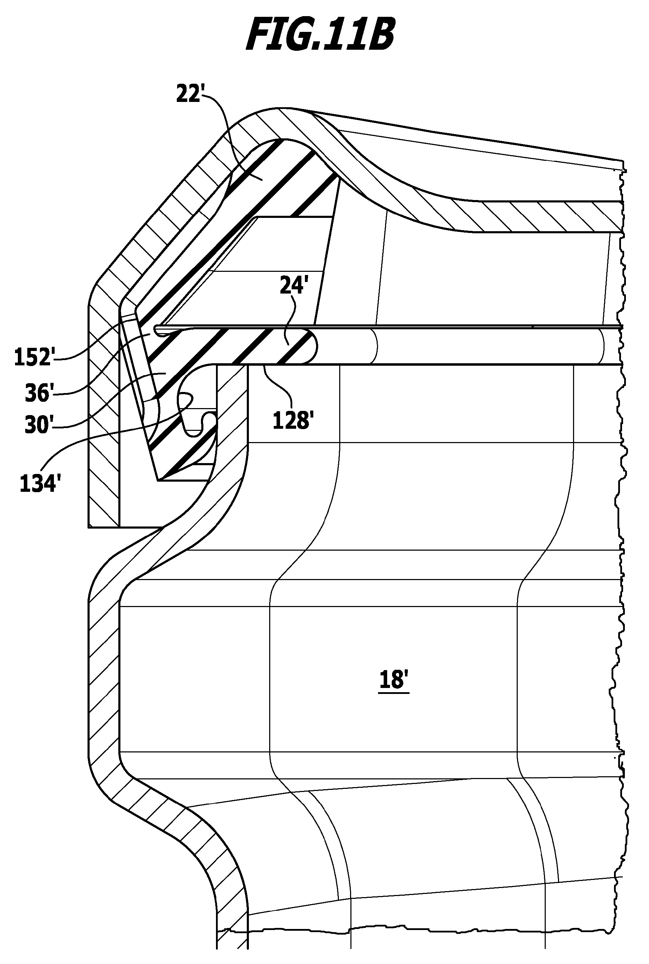

In der Schließstellung des Sterilisierbehälters 10' ist der Dichtlippenträger 30' jedoch, wie in Figur 11B dargestellt, etwas in Richtung auf die Außenseite 66' hin verschwenkt, so dass die Ausnehmung 148' und damit der Überdruckströmungskanal 152' zur Umgebung 20 des Sterilisierbehälters hin geöffnet wird. Herrscht in der Umgebung 20 ein Druck, welcher einen im Behälterinnenraum 18' herrschenden Druck signifikant übersteigt, kann in der Schließstellung ein gasförmiges Medium, beispielsweise Heißdampf, in die Überdruckströmungskanäle 152' eindringen und steht mit dem Umgebungsdruck an dem an der Wand 46' anliegenden oberen Ende 154', welches in Form eines Dichtwulstes 156' ausgebildet ist, an. Aufgrund ihrer Elastizität und/oder Flexibilität der Dichtung 16' kann der Dichtwulst 156' etwas in Richtung auf den Behälterinnenraum 18' von der Wand 46' abheben und öffnet so einen schmalen Strömungspfad 158' zwischen dem Dichtwulst 156' und der Wand 46', so dass ein schneller Druckausgleich zwischen der Umgebung 20 und der Behälterinnenraum 18' möglich ist. Kann ein Druckausgleich zwischen der Umgebung 20 und dem Behälterinnenraum 18' insbesondere bei der Beaufschlagung des geschlossenen Sterilisierbehälters 10' bei der Sterilisation in einem Sterilisator nicht schnell genug erfolgen, beispielsweise wenn ein Sterilfilter und/oder ein entsprechendes Ventil des Sterilisierbehälters 10' nicht den erforderlichen Gasaustausch pro Zeiteinheit ermöglichen, besteht die Gefahr, dass der Sterilisierbehälter 10' zusammengedrückt wird. Durch die Überdruckströmungskanäle 152' in Verbindung mit dem besonders geformten Ende 154' kann diese Gefahr praktisch ausgeräumt werden. Die Dichtung 16' hat somit gleichzeitig auch die Funktion eines Überdruckeinlasssicherheitsventils.

Die Dichtungen 16 und 16' sind beide derart konstruiert, dass sie sowohl an der Stirnkanten 68 beziehungsweise 68' als auch an den Außenseiten 66 beziehungsweise 66' anliegen und so eine doppelte, das heißt redundante Abdichtung des Behälterinnenräume 18 beziehungsweise 18' ermöglichen. Beim Öffnen der Sterilisierbehälter 10, 10' wird das jeweilige Behälteroberteil 14, 14' aufgrund der Elastizität der Dichtungen 16, 16' etwas angehoben, so dass in beschriebener Weise jeweils die zweite Dichtlippe 26, 26' wieder etwas nach außen vom jeweiligen Randbereich 60, 60' wegklappt. Dadurch kann das Behälteroberteil 14, 14' ohne zusätzliche Kraft abgehoben werden. Die besondere Anordnung der Dichtung 16 beziehungsweise 16' am Behälteroberteil 14 beziehungsweise 14' ermöglicht es, die Sterilitätsgrenze an eine äußere Seite des Behälterunterteils 12, 12' zu verlegen und gleichzeitig eine doppelte Dichtung zu realisieren, und zwar ohne einen für eine doppelte Dichtung normalerweise üblichen zusätzlichen Kraftaufwand beim Abheben des Behälteroberteils 14, 14' vom Behälterunterteil 12, 12'. Ferner ermöglichen es die Druckausgleichsbereiche 116 beziehungsweise die Überdruckströmungskanäle 152, einen Druckausgleich bei sehr großen Druckdifferenzen zwischen im Behälterinnenraum 18, 18' und der Umgebung 20 herrschenden Drücken herzustellen. Große Druckdifferenzen treten insbesondere immer dann auf, wenn der Sterilisierbehälter 10, 10' im Sterilisator mit Heißdampf beaufschlagt wird und der Druck in der Umgebung 20 den Druck im Behälterinnenraum 18, 18' zu Beginn des Sterilisationsprozesses deutlich übersteigt.

Die Dichtungen 16 und 16' ist vorzugsweise aus einem Kunststoff hergestellt, insbesondere aus einem elastomeren, dampfsterilisierbaren Kunststoff.

## Patentansprüche

1. Medizinische Dichtung (16; 16') für einen medizinischen Sterilisierbehälter (10; 10'), welcher ein Behälterunterteil (12; 12') und ein Behälteroberteil (14; 14') zum Verschließen des Behälterunterteils (12; 12') umfasst, welche Dichtung (16; 16') in sich geschlossen ausgebildet und umlaufend am Behälteroberteil (14; 14') anordenbar ist, einen Dichtelementhaltekörper (22; 22') zum Festlegen am Behälteroberteil (14; 14') und zwei am Dichtelementhaltekörper (22; 22') angeordnete, umlaufende Dichtlippen (24, 26; 24', 26') umfasst, **dadurch gekennzeichnet, dass** mindestens eine der Dichtlippen (24, 26; 24', 26') gelenkig am Dichtelementhaltekörper (22; 22') gehalten oder gelagert ist, dass die Dichtung einen umlaufenden Dichtlippenträger (30; 30') umfasst, welcher mindestens eine der Dichtlippen (24, 26; 24', 26') trägt oder umfasst und gelenkig mit dem Dichtelementhaltekörper (22; 22') gekoppelt oder verbunden ist, und dass der Dichtelementhaltekörper (22; 22') und der Dichtlippenträger (30; 30') über ein Gelenk (32; 32') miteinander gekoppelt oder verbunden sind.

2. Medizinische Dichtung nach Anspruch 1, **dadurch gekennzeichnet, dass** der Dichtlippenträger (30; 30') verschwenkbar oder im Wesentlichen verschwenkbar am Dichtelementhaltekörper (22; 22') gelagert ist.

3. Medizinische Dichtung nach Anspruch 2, **dadurch gekennzeichnet, dass** der Dichtlippenträger (30; 30') beide Dichtlippen (24, 26; 24', 26') trägt oder umfasst.

4. Medizinische Dichtung nach einem der voranstehenden Ansprüche,
**dadurch gekennzeichnet, dass** der Dichtlippenträger (30; 30') eine Innenseite (134; 134') und eine Außenseite (118; 118') umfasst und dass die beiden Dichtlippen (24, 26; 24', 26') von der Innenseite (134; 134') abstehend angeordnet oder ausgebildet sind.

5. Medizinische Dichtung nach einem der voranstehenden Ansprüche,
**dadurch gekennzeichnet, dass** die Dichtlippen (24, 26; 24', 26') ausgebildet sind zum Anlegen an quer oder im Wesentlichen quer zueinander verlaufende Flächen des Behälterunterteils (12; 12'), vorzugsweise zum Anlegen an senkrecht zueinander verlaufende Flächen des Behälterunterteils (12; 12').

6. Medizinische Dichtung nach einem der voranstehenden Ansprüche,
**dadurch gekennzeichnet, dass** eine erste Dichtlippe (24; 24') ausgebildet ist zum Anlegen an eine vom Behälterunterteil (12; 12') in Richtung auf das Behälteroberteil (14; 14') hin weisende umlaufende obere Kante (68; 68').

7. Medizinische Dichtung nach einem der voranstehenden Ansprüche,
**dadurch gekennzeichnet, dass** eine zweite Dichtlippe (26; 26') ausgebildet ist zum Anlegen an eine vom Behälterunterteil (12; 12') weg weisende Außenfläche (66; 66') desselben.

8. Medizinische Dichtung nach Anspruch 6 oder 7, **dadurch gekennzeichnet, dass** die erste Dichtlippe (24; 24') in einer Grundstellung der Dichtung (16; 16'), in welcher die beiden Dichtlippen (24, 26; 24', 26') relativ zum Dichtelementhaltekörper (22; 22') unausgelenkt sind, schräg nach unten vom Dichtelementhaltekörper (22; 22') weg geneigt ist.

9. Medizinische Dichtung nach einem der voranstehenden Ansprüche,
**dadurch gekennzeichnet, dass** die zweite Dichtlippe (26; 26') in einer Grundstellung, in welcher die beiden Dichtlippen (24, 26; 24', 26') relativ zum Dichtelementhaltekörper (22; 22') unausgelenkt sind, schräg nach oben auf den Dichtelementhaltekörper (22; 22') hin geneigt ist.

10. Medizinische Dichtung nach einem der Ansprüche 5 bis 9, **dadurch gekennzeichnet, dass** der Dichtlippenträger (30) auf seiner Außenseite (118) eine Mehrzahl von Wandabstandshalteelementen (120) umfasst.

11. Medizinische Dichtung nach einem der Ansprüche 6 bis 10, **gekennzeichnet durch** ein Anschlagelement (136; 136'), an welchem die erste Dichtlippe (24; 24') in einer Dichtstellung, in welcher die Dichtlippen (24, 26; 24', 26') relativ zum Dichtelementhaltekörper aus der Grundstellung ausgelenkt sind, anschlägt.

12. Medizinische Dichtung nach einem der voranstehenden Ansprüche,
**gekennzeichnet durch** eine Mehrzahl von Befestigungselementaufnahmen (70; 70') zum Aufnehmen jeweils eines Befestigungselements (72; 72') zum Festlegen der Dichtung (16; 16') am Behälteroberteil (14; 14'), welche Befestigungselementaufnahmen (70; 70') vorzugsweise in Form von Durchbrechungen (74), Ausnehmungen (142') oder Sacklöchern (143') des Dichtelementhaltekörpers (22; 22') ausgebildet sind.

13. Medizinische Dichtung nach einem der voranstehenden Ansprüche,
**dadurch gekennzeichnet, dass** am Dichtelementhaltekörper (22') und/oder am Dichtlippenträger (30') auf von den Dichtlippen (24, 26) weg weisenden Außenflächen (38', 118') Ausnehmungen ausgebildet sind zur Ausbildung eines Überdruckströmungskanals (152').

14. Medizinischer Sterilisierbehälter (10; 10') mit einem wannenförmigen Behälterunterteil (12; 12') und einem Behälteroberteil (14; 14') zum Verschließen des Behälterunterteils (12; 12') in einer Verschlussstellung, welches Behälteroberteil (14; 14') eine medizinische Dichtung (16; 16') umfasst, welche Dichtung (16; 16') in sich geschlossen ausgebildet und umlaufend am Behälteroberteil (14; 14') angeordnet ist, einen am Behälteroberteil (14; 14') festgelegten Dichtelementhaltekörper (22; 22') und zwei am Dichtelementhaltekörper (22; 22') angeordnete, umlaufende Dichtlippen (24, 26; 24', 26') umfasst, **dadurch gekennzeichnet, dass** die medizinische Dichtung (16; 16') in Form einer medizinischen Dichtung (16; 16') nach einem der voranstehenden Ansprüche ausgebildet ist.

15. Medizinischer Sterilisierbehälter nach Anspruch 14, **dadurch gekennzeichnet, dass** an der Dichtung (16') mindestens ein Überdruckströmungskanal (152') ausgebildet ist, welcher in der Grundstellung der Dichtung (16') geschlossen und in der Schließstellung zur Umgebung (20) des Sterilisierbehälters (10') hin geöffnet ist.

## Claims

1. Medical seal (16; 16') for a medical sterilizing container (10; 10') having a container bottom part (12; 12') and a container top part (14; 14') for closing the container bottom part (12; 12'), said seal (16; 16') being formed so as to be closed within itself and being adapted to be arranged on and extend around the container top part (14; 14') and comprising a sealing element holder body (22; 22') for fixation to the container top part (14; 14') and two sealing lips (24, 26; 24', 26') arranged on and extending around the sealing element holder body (22; 22'), at least one of the sealing lips (24, 26; 24', 26') being held or mounted in an articulated manner on the sealing element holder body (22; 22'), **characterized in that** the seal comprises a circumferential sealing lip carrier (30; 30') which carries or comprises at least one of the sealing lips (24, 26; 24', 26') and is coupled or connected in an articulated manner to the sealing element holder body (22; 22'), and **in that** the sealing element holder body (22; 22') and the sealing lip carrier (30; 30') are coupled or connected to each other by a joint (32; 32').

2. Medical seal in accordance with claim 1, **characterized in that** the sealing lip carrier (30; 30') is mounted for pivotal or substantially pivotal movement on the sealing element holder body (22; 22').

3. Medical seal in accordance with claim 2, **characterized in that** the sealing lip carrier (30; 30') carries or comprises both sealing lips (24, 26; 24', 26').

4. Medical seal in accordance with any one of the preceding claims, **characterized in that** the sealing lip carrier (30; 30') has an inner side (134; 134') and an outer side (118; 118'), and **in that** the two sealing lips (24, 26; 24', 26') are arranged or formed so as to protrude from the inner side (134; 134').

5. Medical seal in accordance with any one of the preceding claims, **characterized in that** the sealing lips (24, 26; 24', 26') are formed for placement on surfaces of the container bottom part (12; 12') extending transversely or substantially transversely to each other, preferably for placement on surfaces of the container bottom part (12; 12') extending perpendicularly to each other.

6. Medical seal in accordance with any one of the preceding claims, **characterized in that** a first sealing lip (24, 24') is formed for placement on a circumferential upper edge (68, 68') facing from the container bottom part (12; 12') in the direction towards the container top part (14; 14').

7. Medical seal in accordance with any one of the preceding claims, **characterized in that** a second sealing lip (26; 26') is formed for placement on an outer surface (66; 66') of the container bottom part (12; 12') facing away from said container bottom part (12; 12').

8. Medical seal in accordance with claim 6 or 7, **characterized in that** when the seal (16; 16') is in an original position in which the two sealing lips (24, 26; 24', 26') are undeflected relative to the sealing element holder body (22; 22'), the first sealing lip (24, 24') is inclined downwards away from the sealing element holder body (22; 22').

9. Medical seal in accordance with any one of the preceding claims, **characterized in that** in an original position in which the two sealing lips (24, 26; 24', 26') are undeflected relative to the sealing element holder body (22; 22'), the second sealing lip (26; 26') is inclined upwards towards the sealing element holder body (22; 22').

10. Medical seal in accordance with any one of claims 5 to 9, **characterized in that** the sealing lip carrier (30) has a plurality of wall spacer elements (120) on its outer side (118).

11. Medical seal in accordance with any one of claims 6 to 10, **characterized by** a stop element (136; 136') which the first sealing lip (24; 24') strikes in a sealing position in which the sealing lips (24, 26; 24', 26') are deflected from the original position relative to the sealing element holder body.

12. Medical seal in accordance with any one of the preceding claims, **characterized by** a plurality of fastening element receptacles (70; 70') for receiving one fastening element (72; 72') each for fixing the seal (16; 16') to the container top part (14; 14'), said fastening element receptacles (70; 70') preferably being in the form of through-openings (74), recesses (142') or blind holes (143') of the sealing element holder body (22; 22').

13. Medical seal in accordance with any one of the preceding claims, **characterized in that** recesses for formation of an overpressure flow channel (152') are formed on the sealing element holder body (22') and/or on the sealing lip carrier (30') on outer surfaces (38'; 118') facing away from the sealing lips (24, 26).

14. Medical sterilizing container (10; 10') having a tub-shaped container bottom part (12; 12') and a container top part (14; 14') for closing the container bottom part (12; 12') in a closed position, said container top part (14; 14') comprising a medical seal (16; 16'), said seal (16; 16') being formed so as to be closed within itself and being arranged on and extending around the container top part (14; 14') and comprising a sealing element holder body (22; 22') fixed to the container top part (14; 14') and two sealing lips (24, 26; 24', 26') arranged on and extending around the sealing element holder body (22; 22'), **characterized by** a medical seal (16; 16') in accordance with any one of the preceding claims.

15. Medical sterilizing container in accordance with claim 14, **characterized in that** there is formed on the seal (16') at least one overpressure flow channel (152') which, when the seal (16') is in the original position, is closed and, in the closed position, is open towards the environment (20) of the sterilizing container (10').

## Revendications

1. Joint d'étanchéité médical (16 ; 16') pour un récipient médical stérilisable (10 ; 10'), qui comprend une partie inférieure de récipient (12 ; 12') et une partie supérieure de récipient (14 ; 14') pour la fermeture de la partie inférieure de récipient (12 ; 12'), ce joint d'étanchéité (16 ; 16') étant conçu de manière refermée sur lui-même pouvant être disposé de manière circulaire sur la partie supérieure de récipient (14 ; 14'), un corps de maintien d'élément d'étanchéité (22 ; 22') destiné à être fixé sur la partir supérieure du récipient (14 ; 14') et deux lèvres d'étanchéité (24, 26 ; 24', 26') circulaires disposées sur le corps de maintien d'élément d'étanchéité (22 ; 22'), **caractérisé en ce qu'**au moins une des lèvres d'étanchéité (24, 26 ; 24', 26') est maintenue ou logée de manière articulée au niveau du corps de maintien d'élément d'étanchéité (22 ; 22'), **en ce que** le joint d'étanchéité comprend un support de lèvre d'étanchéité (30 ; 30') qui supporte ou comprend au moins une des lèvres d'étanchéité (24, 26 ; 24', 26') est couplé ou relié de manière articulée avec le corps de maintien d'élément d'étanchéité (22 ; 22') et **en ce que** le corps de maintien d'élément d'étanchéité (22 ; 22') et le support de lèvre d'étanchéité (30 ; 30') sont couplés ou reliés entre eux par l'intermédiaire d'une articulation (32 ; 32').

2. Joint d'étanchéité médical selon la revendication 1, **caractérisé en ce que** le support de lèvre d'étanchéité (30 ; 30') est logé de manière pivotante ou globalement pivotante au niveau du corps de maintien d'élément d'étanchéité (22 ; 22').

3. Joint d'étanchéité médical selon la revendication 2, **caractérisé en ce que** le support de lèvre d'étanchéité (30 ; 30') supporte ou comprend les deux lèvres d'étanchéité (24, 26 ; 24', 26').

4. Joint d'étanchéité médial selon l'une des revendications précédentes, **caractérisé en ce que** le support de lèvre d'étanchéité (30 ; 30') comprend un côté intérieur (134 ; 134') et un côté extérieur (118 ; 118') et **en ce que** les deux lèvres d'étanchéité (24, 26 ; 24', 26') sont disposées ou réalisées à une certaine distance du côté intérieur (134 ; 134').

5. Joint d'étanchéité médial selon l'une des revendications précédentes, **caractérisé en ce que** les lèvres d'étanchéité (24, 26 ; 24', 26') sont conçues pour s'appuyer sur des surfaces, s'étendant transversalement ou globalement transversalement entre elles, de la partie inférieure du récipient (12 ; 12'), de préférence pour s'appuyer contre des surfaces, s'étendant perpendiculairement entre elles, de la partie inférieure du récipient (12 ; 12').

6. Joint d'étanchéité médial selon l'une des revendications précédentes, **caractérisé en ce qu'**une première lèvre d'étanchéité (24 ; 24') est conçue pour s'appuyer contre une arête supérieure circulaire (68 ; 68') orientée de la partie inférieure du récipient (12 ; 12') en direction de la partie supérieure du récipient (14 ; 14').

7. Joint d'étanchéité médial selon l'une des revendications précédentes, **caractérisé en ce qu'**une deuxième lèvre d'étanchéité (26 ; 26') est conçue pour s'appuyer contre une surface extérieure (66 ; 66') de la partie inférieure du récipient s'éloignant de la partie inférieure du récipient (12 ; 12').

8. Joint d'étanchéité médial selon la revendication 6 ou 7, **caractérisé en ce que**, dans une position de base du joint d'étanchéité (16 ; 16'), dans laquelle les deux lèvres d'étanchéité (24, 26 ; 24', 26') ne sont pas déviées par rapport au corps de maintien d'élément d'étanchéité (22 ; 22'), la première lèvre d'étanchéité (24 ; 24') est inclinée obliquement vers le bas en s'éloignant du corps de maintien d'élément d'étanchéité (22 ; 22').

9. Joint d'étanchéité médial selon l'une des revendications précédentes, **caractérisé en ce que**, dans une position de base, dans laquelle les deux lèvres d'étanchéité (24, 26 ; 24', 26') ne sont pas déviées par rapport au corps de maintien d'élément d'étanchéité (22 ; 22'), la deuxième lèvre d'étanchéité (26 ; 26') est inclinée obliquement vers le haut en direction du corps de maintien d'élément d'étanchéité (22 ; 22').

10. Joint d'étanchéité médial selon l'une des revendications 5 à 9, **caractérisé en ce que** le support de lèvre d'étanchéité (30) comprend, sur son côté extérieur (118), une pluralité d'éléments d'espacement de parois (120).

11. Joint d'étanchéité médial selon l'une des revendications 6 à 10, **caractérisé par** un élément de butée (136 ; 136'), contre lequel la première lèvre d'étanchéité (24 ; 24') s'appuie dans une position d'étanchéité dans laquelle les lèvres d'étanchéité (24, 26 ; 24', 26') sont déviées par rapport au corps de maintien d'élément d'étanchéité hors de la position de base.

12. Joint d'étanchéité médial selon l'une des revendications précédentes, **caractérisé par** une pluralité de logements d'éléments de fixation (70 ; 70') pour le logement d'un élément de fixation (72 ; 72') pour la fixation du joint d'étanchéité (16 ; 16') à la partie supérieure du récipient (14 ; 14'), ces logements d'éléments de fixation (70 ; 70') se présentant de préférence sous la forme de découpes (74), d'évidements (142') ou de trous borgnes (143') du corps de maintien d'élément d'étanchéité (22 ; 22').

13. Joint d'étanchéité médial selon l'une des revendications précédentes, **caractérisé en ce que**, sur le corps de maintien d'élément d'étanchéité (22') ou sur le support de lèvre d'étanchéité (30'), sur des surfaces extérieures (38', 118') opposées aux lèvres d'étanchéité (24, 26), sont réalisés des évidements permettant de former un canal d'écoulement à surpression (152').

14. Récipient stérilisable (10 ; 10') avec une partie inférieure de récipient (12 ; 12') en forme de bac et une partie supérieure de récipient (14 ; 14') pour la fermeture de la partie inférieure du récipient (12 ; 12') dans une position de fermeture, cette partie supérieure de récipient (14 ; 14') comprenant un joint d'étanchéité médical (16 ; 16'), ce joint d'étanchéité (16 ; 16') étant conçu de manière refermée sur lui-même et étant disposé de manière circulaire sur la partie supérieure du récipient (14 ; 14'), un corps de maintien d'élément d'étanchéité (22 ; 22') fixé sur la partie supérieure du récipient (14 ; 14') et deux lèvres d'étanchéité (24, 26 ; 24', 26') circulaires disposées sur le corps de maintien d'élément d'étanchéité (22 ; 22'), **caractérisé en ce que** le joint d'étanchéité médical (16 ; 16') se présente sous la forme d'un joint d'étanchéité médical (16 ; 16') selon l'une des revendications précédentes.

15. Récipient médical stérilisable selon la revendication 14, **caractérisé en ce que**, sur le joint d'étanchéité (16'), se trouve au moins un canal d'écoulement de surpression (152'), qui est fermé dans la position de base du joint d'étanchéité (16') et qui est ouvert en direction de l'environnement (20) du récipient stérilisable (10') dans la position de femreture.
